# EUROPÄISCHE PATENTSCHRIFT

(11) **EP 3 106 188 B1**
(45) Veröffentlichungstag und Bekanntmachung des Hinweises auf die Patenterteilung: **06.02.2019**
(21) Anmeldenummer: 15172166.9
(22) Anmeldetag: 15.06.2015
(51) Int. Cl.: A61M 5/00, A61M 5/32, A61M 5/31

(54) **VERFAHREN ZUR MONTAGE EINER SICHERHEITSVORRICHTUNG AUF EINEM SPRITZENKÖRPER**
METHOD FOR MOUNTING A SAFETY DEVICE ON A SYRINGE
PROCÉDÉ DE MONTAGE D'UN DISPOSITIF DE SÉCURITÉ SUR UNE SERINGUE

(43) Veröffentlichungstag der Anmeldung: 21.12.2016
(73) Patentinhaber: Gerresheimer Regensburg GmbH, 93047 Regensburg (DE); Gerresheimer Bünde GmbH, 32257 Bünde (DE)
(72) Erfinder: Wittland, Frank, 32257 Bünde (DE); Vogl, Maximilian, 92708 Mantel (DE)
(74) Vertreter: Hannke, Christian

(56) Entgegenhaltungen:
- EP-A1- 1 818 069
- WO-A1-2011/110872
- WO-A1-2015/022787
- DE-U1- 29 820 166

## Beschreibung

Die Erfindung betrifft ein Verfahren zur Montage einer Sicherheitseinrichtung und Montage der Sicherheitseinrichtung auf einem Spritzenkörper.

Aus dem Stand der Technik sind bisher Sicherheitseinrichtungen zur Vermeidung von Stichverletzungen bekannt, die nach einem Befüllen der Spritzen, beispielsweise von einem Zulieferer unter Reinraumbedingungen in einem Spritzennest oder dergleichen, um die gesamte Spritze herum montiert werden.

Diese Sicherheitsvorrichtungen vergrößern jedoch die Dimension, sowohl in der reinen Größe als auch dem Gewicht, der Spritze zum Gebrauch, so dass eine Handhabung von Spritzen mit derartigen Sicherheitsvorrichtungen erheblich erschwert wird. Darüber hinaus werden die Kosten durch den erhöhten Materialeinsatz ebenso erhöht und die Herstellungsdauer einer Spritze mit einer Sicherheitseinrichtung, da die Sicherheitseinrichtung in mehreren Schritten auf der Spritze zusammengebaut werden muss.

Darüber hinaus ist es mit einer derartigen Konstruktion der Sicherheitseinrichtung nicht möglich, die Spritze samt Sicherheitseinrichtung in ein Spritzennest einzuhängen und zu befüllen, da das Spritzennest einer DIN-Norm entspricht und somit eine genormte Größe aufweist. Daher muss die Spritze erst befüllt werden, bevor die Spritze mit der Sicherheitseinrichtung versehen werden kann. Dabei entsteht natürlich auch das Risiko, sich mit einem Arzneimittel, das in die Spritze eingefüllt wurde, zu injizieren.

Darüber hinaus weist die nachträgliche Aufbringung den Nachteil auf, dass die bereits befüllte Spritze beim Aufbau der Sicherheitseinrichtung auf die Spritze beschädigt werden kann und so unbrauchbar ist. Dies ist insbesondere der Fall, wenn die Spritze aus Glas oder einem anderen zerbrechlichen Material besteht. Wenn die Spritze beschädigt wird, so ist auch das enthaltene Arzneimittel nicht mehr verwendbar, wodurch erhöhte Kosten bei der Herstellung entstehen können.

Die WO 2015/022787 A1 offenbart ein Verfahren zur Montage einer Sicherheitseinrichtung und Montage der Sicherheitseinrichtung auf einem Spritzenkörper mit den Merkmalen des einleitenden Teils des Anspruchs 1.

Es ist daher die Aufgabe der vorliegenden Anmeldung, ein Verfahren zur Montage einer Sicherheitseinrichtung bereitzustellen, so dass die Sicherheitseinrichtung vor einem Abfüllprozess des Spritzenkörpers auf dem Spritzenkörper montiert werden kann, insbesondere in einem einzigen Schritt auf dem Spritzenkörper montiert werden kann.

Gelöst wird diese zugrunde liegende Aufgabe von einem Verfahren zur Montage einer Sicherheitseinrichtung und Montage der Sicherheitseinrichtung auf einem Spritzenkörper vor einem Abfüllprozess des Spritzenkörpers gemäß Anspruch 1.

Erfindungsgemäß wird demnach wie vorliegend zunächst die Sicherheitseinrichtung aus mehreren Einzelteilen, nämlich der Hülse, dem Federelement und dem Montageelement, zusammengebaut und anschließend als Einheit in einem einzigen Schritt auf dem Spritzenkörper montiert, vorliegend durch das Verbinden des Montageelements mit dem Spritzenkörper.

Die Montagerichtung erstreckt sich hierbei von einem proximalen Ende der Hülse zu einem distalen Ende der Hülse hin. Das Federelement sowie das Montageelement werden also an dem proximalen Ende der Hülse eingebracht und in Richtung des distalen Endes verschoben, wobei vorzugsweise im Inneren der Hülse ein Auflagebereich angeordnet ist, an dem zumindest das Federelement durch das Einführen des Federelements in das Innere der Hülse wirkverbunden ist und so an dem Auflagebereich anliegt.

Ebenso wie das Federelement wird das Montageelement in das Innere der Hülse eingebracht. Das Montageelement ist dabei vorzugsweise mit der Hülse verbindbar, so dass das Federelement einerseits durch den Auflagebereich und andererseits durch das Montageelement in der Hülse gehalten wird. Das Federelement ist also so gegen ein Herausfallen aus der Hülse gesichert.

Wenn ein Benutzer der Spritze nun den Spritzenkörper gegenüber der Sicherheitseinrichtung bewegt, so wirkt das Federelement dieser Bewegung entgegen und würde, falls die Relativbewegung unterbunden wird, den Spritzenkörper bezüglich der Sicherheitseinrichtung wieder zurückführen.

Durch das Federelement kann also gewährleistet werden, dass die Nadel nach Gebrauch der Spritze sicher in die Sicherheitseinrichtung, vorzugsweise automatisch, rückgeführt wird. Dabei kann das Federelement auf verschiedene Weisen ausgestaltet sein. Vorzugsweise handelt es sich bei dem Federelement um eine Spiralfeder.

Gemäß einer bevorzugten Ausführungsform wird dabei das Montageelement mit dem Spritzenkörper durch Aufstecken des Montageelements auf dem Spritzenkörper verbunden. Gemäß einer weiter bevorzugten Ausführungsform wird das Montageelement auf dem Spritzenkörper aufgeclipst, wodurch eine besonders einfache und sichere Montage der Sicherheitseinrichtung auf dem Spritzenkörper gewährleistet werden kann.

Das Montageelement umfasst zur Montage vorzugsweise eine Montageöffnung, in welcher gegebenenfalls ein oder mehrere Rastelemente zum Aufclipsen des Montageelements und demzufolge der Sicherheitseinrichtung angeordnet sind.

Erfindungsgemäß wird das Montageelement mit der Hülse mittels mindestens eines Führungsstifts verbunden, wobei der Führungsstift auf dem Montageelement angeordnet ist.

Erfindungsgemäß weist hierbei die Hülse eine oder mehrere Ausnehmungen auf, die eine Führungskulisse ausbilden, wobei der Führungsstift in die Ausnehmung bzw. die Führungskulisse eingebracht wird und somit das Montageelement mit der Hülse verbunden wird. Durch die Führungskulisse wird der Führungsstift bei einer Relativbewegung von Spritzenkörper zu Sicherheitseinrichtung geführt. Hierzu bewegt sich das Montageelement erfindungsgemäß radial, also rotierend um eine Achse des Spritzenkörpers, jedoch bewegt es sich nicht in axialer Richtung. Dies wird beispielhaft gelöst durch ein Aufclipsen des Montageelements auf dem Spritzenkörper, wie zuvor beschrieben.

Die Hülse umfasst erfindungsgemäß eine Führungskulisse in Form einer Ausnehmung, in der der Führungsstift hineingebracht wird und somit die Hülse und das Montageelement miteinander verbindet. Die Abschrägung und Aussparung ist also dafür geeignet, den Führungsstift des Montageelements geführt in das Innere der Hülse einzubringen, was eine Montage der Sicherheitseinrichtung erleichtert und beschleunigt.

Die Sicherheit der Sicherheitseinrichtung kann noch weiter verbessert werden, wenn zusätzlich zu der Hülse, dem Federelement und dem Montageelement eine Baugruppe umfassend eine Kappe und ein Nadelschutzteil bereitgestellt wird. Durch diese Baugruppe wird das Stechelement, beispielsweise eine Nadel, noch besser geschützt, insbesondere vor Verwendung der Spritze für eine Injektion. Vorteilhaft kann die Baugruppe vor Gebrauch der Spritze von der Hülse abgezogen werden.

Gemäß einer bevorzugten Ausführungsform wird die Baugruppe mit der Hülse durch Aufschieben der Kappe auf der Hülse entgegen der Montageeinrichtung verbunden, wodurch eine besonders einfach und schnelle Montage der Baugruppe auf der Hülse gewährleistet ist.

Vorteilhaft umfasst die Hülse Ausnehmungen, die komplementär zu der Kappe der Baugruppe ausgebildet sind, so dass hierdurch schon eine kraftschlüssige Verbindung durch das Aufschieben der Kappe auf der Hülse hergestellt werden kann.

Gemäß einer weiter bevorzugten Ausführungsform wird der Spritzenkörper mit der darauf montierten Sicherheitseinrichtung sterilisiert. Somit kann durch die Vormontage der Sicherheitseinrichtung auf dem Spritzenkörper die gesamte Anordnung bestehend aus in einem Schritt sterilisiert werden, wodurch dadurch mehrere Sterilisationsschritte, wie es bei dem Stand der Technik durch nachträgliche Montage der Sicherheitseinrichtung der Fall ist, überflüssig und können weggelassen werden, wodurch die Herstellungsgeschwindigkeit einer Spritze und die Anlagenkomplexität verringert werden kann.

Erfindungsgemäß wird der Spritzenkörper mit der montierten Sicherheitseinrichtung in ein Spritzennest eingehängt. Vorzugsweise wird das Spritzennest umfassend den Spritzenkörper mit Sicherheitseinrichtung in eine Wanne eingehängt, so dass das Spritzennest sicher transportiert werden kann, beispielsweise zu einem Unternehmen, der die Spritzen befüllt. Dabei ist es auch möglich, die Wanne mit Spritzennest und Spritze vorzugsweise unter Reinraumbedingungen mit Hilfe einer Abdeckung, beispielsweise einer Folie, zu verschließen.

Weitere vorteilhafte Ausführungsformen ergeben sich aus den Unteransprüchen.

Weitere Ziele, Vorteile und Zweckmäßigkeiten der vorliegenden Erfindung sind der nachfolgenden von der Beschreibung in Verbindung mit der Zeichnung zu entnehmen. Hierbei zeigen:
- Fig. 1: einen Ausschnitt des Spritzenkörpers;
- Fig. 2A: eine perspektivische Ansicht der Hülse von vorne;
- Fig. 2B: eine perspektivische Ansicht der Hülse von hinten;
- Fig. 2C: eine perspektivische Ansicht der Kappe;
- Fig. 2D: die Montage der Kappe auf der Hülse;
- Fig. 3A: eine perspektivische Ansicht des Montageelements von oben;
- Fig. 3B: eine perspektivische Ansicht des Montageelements von unten;
- Fig. 4: einen Spritzenkörper mit darauf montierten Sicherheitseinrichtung in einem Längsschnitt;
- Fig. 5: ein Spritzennest;
- Fig. 6: in ein Spritzennest eingehängte Spritzen umfassend Spritzenkörper und Sicherheitseinrichtung
- Fig. 7: Flussdiagramm zur Darstellung der einzelnen Verfahrensschritte gemäß einer Ausführungsform;

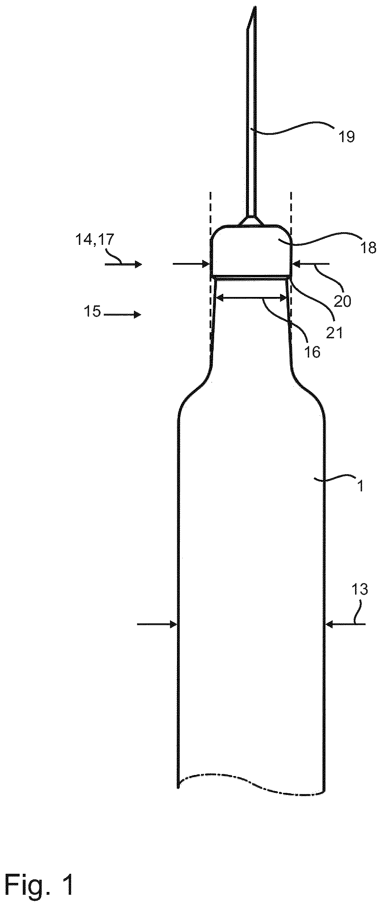

Figur 1 zeigt hierbei einen möglichen der Erfindung zugrunde liegenden Spritzenkörper 1. Handelsübliche Spritzenkörper 1 sind dabei im Wesentlichen zylinderförmig ausgestaltet, wie auch der vorliegende Spritzenkörper 1, und weist einen vorbestimmten bzw. vorbestimmbaren Durchmesser 13 des auf. Vorliegend weist darüber hinaus der Spritzenkörper 1 an seinem distalen Ende 14 einen verengten Bereich 15 auf, der ebenfalls im Wesentlichen zylinderförmig ausgestaltet ist. Vorliegend weist jedoch der verengte Bereich 15 eine Kegelstumpfform auf, wobei der verengte Bereich 15 in der Form eines Kegelstumpfes einen Durchmesser 16 an der Deckfläche (hier nicht gezeigt), also dem distalen Ende 17 des verengten Bereichs 15, auf, wobei der Durchmesser 16 des verengten Bereichs 15 kleiner ist als der Durchmesser 13 des Spritzenkörpers 1.

Weiter ist vorliegend an dem distalen Ende 17 des verengten Bereichs 15 eine Nadelhalterung 18 mit einem Stechelement 19, vorliegend einer Nadel 19, angeordnet, wobei diese Nadelhalterung 18 einen Durchmesser 20 aufweist, der größer ist als der Durchmesser 16 des verengten Bereichs 15, jedoch kleiner ist als der Durchmesser 13 des Spritzenkörpers 1. Weiter ist die Nadelhalterung 18 an dem distalen Ende 17 des verengten Bereichs 15 angeordnet, so dass ein Übergangsbereich 21 entsteht, wobei durch den Übergangsbereich 21 ein Bereich ausgebildet ist, an dem einerseits der Durchmesser 16 des verengten Bereichs 15 und der Durchmesser 20 der Nadelhalterung aufeinandertreffen und so der Durchmesser an sich sprunghaft verändert wird. Durch den Übergangsbereich 21 ist demnach eine Art Vorsprung ausgebildet.

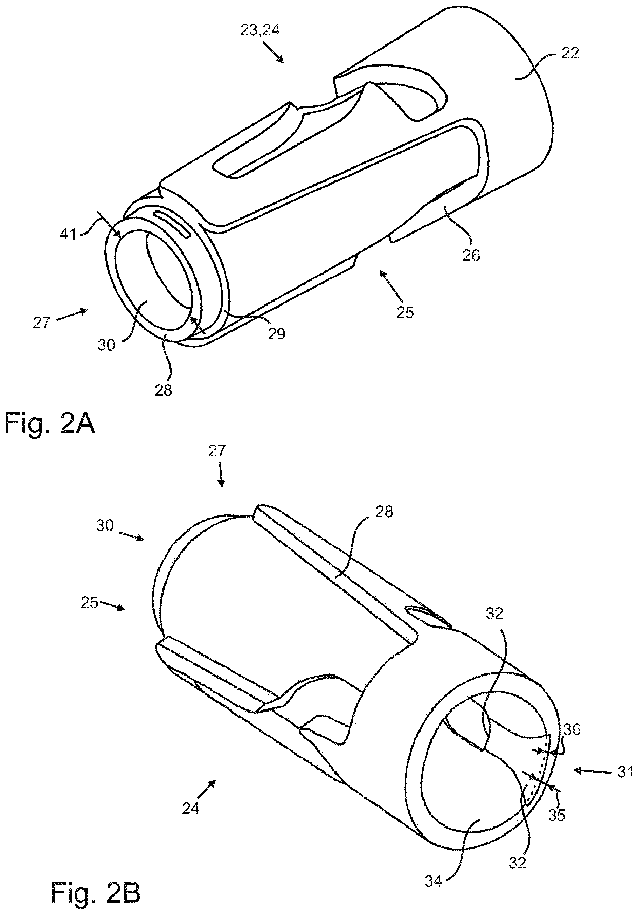

In der Figur 2A wird ein erster wichtiger Bestandteil der Sicherheitseinrichtung 2 in einer perspektivischen Ansicht gezeigt, nämlich die Hülse 22. Wie gut aus der Figur 2A zu erkennen ist, weist die Hülse eine Ausnehmung 24 auf, die die Führungskulisse 23 zur Führung des Führungsstiftes 7 (hier nicht gezeigt) ausbildet. Zusätzlich kann die Hülse 22 einen Ausnahmebereich 25, der durch eine Berandung 26 begrenzt ist, aufweisen. Vorteilhaft ist der Ausnehmung 24 eine spiegelbildliche Ausnehmung 24' sowie dem Ausnahmebereich 25 ein spiegelbildlicher Ausnahmebereich 25' auf der Hülse 22 gegenüberliegend angeordnet.

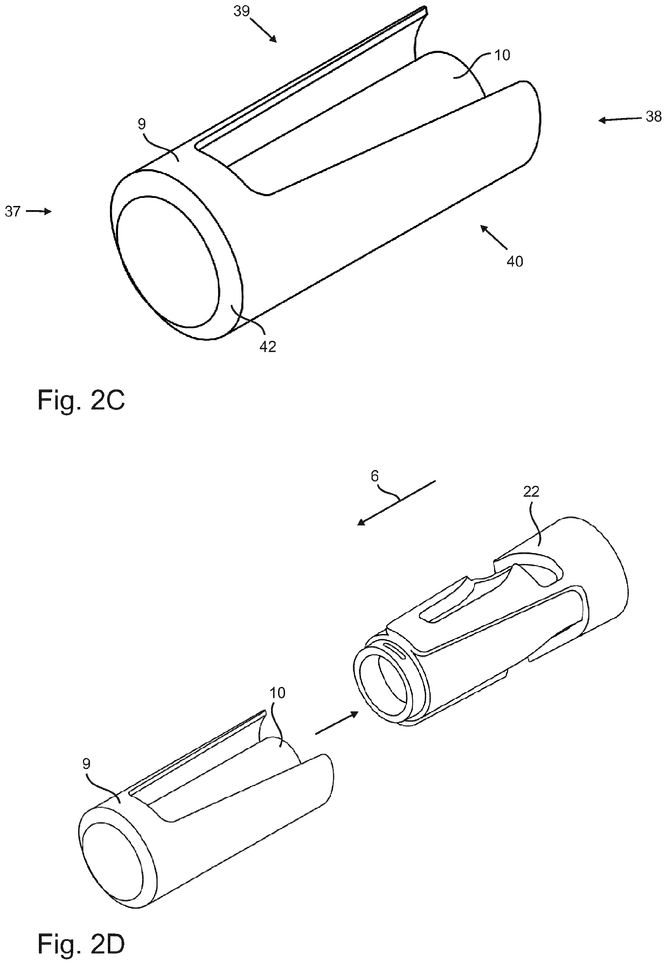

Der Ausnahmebereich 25 dient vorliegend dazu, dass eine Kappe 9 (siehe Fig. 2C) zur Sicherheitserhöhung der Sicherheitseinrichtung 2 geführt aufsteckbar ist. Durch die Berandung 26 des Ausnahmebereichs 25 ist die Kappe 9 mit der Hülse 22 zumindest in Wirkkontakt und bevorzugt zumindest kraftschlüssig verbunden.

Weiter umfasst die Hülse 22 an ihrem distalen Ende 27 einen Kreisring 28 und einen ringförmigen Bereich 29, der in radialer Richtung gesehen um den Kreisring 28 herum angeordnet ist. Auf den Kreisring 28 wird die Kappe 9 aufgesteckt, so dass zusätzlich zu dem Ausnahmebereich 25 eine weitere kraftschlüssige Verbindung zwischen Kappe 9 und Hülse 22 hergestellt wird.

In der Figur 2B ist die Hülse 22 nochmals dargestellt, jedoch in einer anderen Perspektive als in der Figur 2A. Zu sehen ist in der Figur 2B das proximale Ende 31 der Hülse 22, die zumindest einen schrägen Abschnitt 32 aufweist. Vorzugsweise ist die Anzahl der schrägen Abschnitte 32 gleich der Anzahl der Führungsstifte 7 und sind komplementär zu den Führungsstiften 7 auf der Hülse 22 ausgebildet.

Durch die schrägen Abschnitte 32 wird eine Führung der Führungsstifte 7 beim Zusammenbau der Sicherheitseinrichtung 2 dargestellt, wobei die schrägen Abschnitte eine Vertiefung der Hülseninnenwand 34 darstellen, so dass eine Wandstärke 35 der Hülse 22 durch den Betrag 36 der Vertiefung 32 erniedrigt wurde, wodurch ein Einführen des Montageelements 5 mit dem Führungsstift 7 in die Hülse 22 hinein erleichtert wird.

Die Figur 2C zeigt hierbei eine Kappe 9, an der zugleich ein Nadelschutzteil 10 angeordnet ist. Die Kappe 9 umfasst dabei ein erstes Flügelelement 39 und ein zweites Flügelelement 40, die derart ausgestaltet sind, dass sie komplementär zu den Ausnahmebereichen 25 sind. Dabei ist der Außendurchmesser (hier nicht gezeigt) des Nadelschutzteils 10 kleiner als ein Innendurchmesser 41 der Austrittsöffnung 30 bzw. der Hülse 22, wodurch gewährleistet wird, dass das Nadelschutzteil 10 in die Hülse 22 einbringbar ist.

Die Figur 2D zeigt vorliegend, wie die Kappe 9 inklusive Nadelschutzteil 10 auf die Hülse 22 entgegen der Montagerichtung 6, die von dem proximalen Ende 31 der Hülse zu dem distalen Ende 27 entlang einer Längsrichtung der Hülse 22 verläuft, aufgeschoben wird. Nach der Montage ist ein kreisförmiger Bereich 42 der Kappe 9 vorzugsweise in Kontakt mit dem Kreisring 28. Es ist jedoch auch denkbar, dass der kreisförmige Bereich 42 mit dem ringförmigen Bereich 29 in Kontakt steht

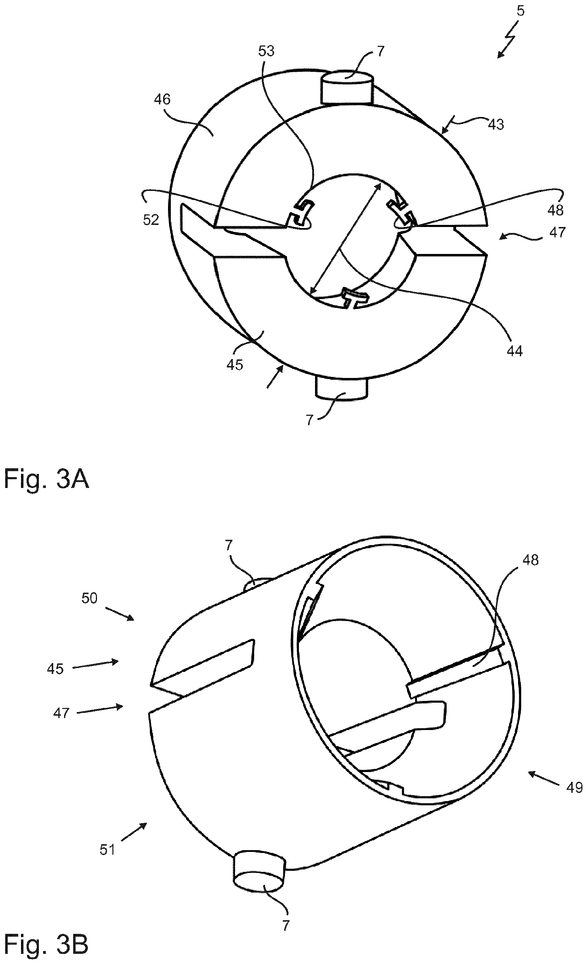

Die Figuren 3A und 3B zeigen das Montageelement 5, mittels welchem die Hülse 2 und somit die Sicherheitseinrichtung 2 im Ganzen mit dem Spritzenkörper 1 verbindbar ist, wobei die Figur 3A das Montageelement 5 in einer perspektivischen Ansicht von oben und die Figur 3B das Montageelement 5 in einer perspektivischen Ansicht von unten zeigt.

Das Montageelement 5 ist vorliegend im Wesentlichen eine zylindrische Form mit einem Außendurchmesser 43 und einem Innendurchmesser 44 auf, wobei durch das Bezugszeichen 45 das distale Ende des Montageelements 5 bezeichnet ist.

Vorliegend sind auf einer Mantelfläche 46 des Montageelements 5 zwei Führungsstifte 7 angeordnet, die sich bezüglich des Montageelements 5 gegenüber liegen. Diese Führungsstifte 7 sind dann in der Führungskulisse 23 der Hülse anordenbar und mit dieser wirkverbindbar.

Zusätzlich weist das Montageelement 5 eine oder mehrere, vorliegend zwei, Aussparungen 47 und ein oder mehrere, vorliegend drei, Rastelemente 48 auf, deren Funktionen nachstehend mit Bezug auf die Figur 3B dargestellt werden.

In der Figur 3B ist mit dem Bezugszeichen 49 ein proximales Ende 49 des Montageelements 5 bezeichnet. Wie zu erkennen ist, verlaufen die Rastelemente 48 des Montageelements 5 ansteigend vom proximalen Ende 49 zum distalen Ende 45 hin, d.h. die Rastelemente 48 sind am distalen Ende 45 in radialer Richtung gesehen größer als am proximalen Ende 49.

Wird beim Zusammenfügen des Montageelements 5 mit dem Spritzenkörper 1, insbesondere mit dem verengten Bereich 15, das Montageelement 5 mit seinem proximalen Ende 49 zuerst auf die Nadelhalterung 18 geschoben, so wird durch den Dickenunterschied in radialer Richtung und ansteigend in axialer Richtung eine erste Hälfte 50 und eine zweite Hälfte 51 des Montageelements 5, die durch die Aussparungen 47 getrennt sind, voneinander wegbewegt. Es ist hierbei also erforderlich, dass das Montageelement 5 zumindest teilweise elastisch ausgebildet ist.

Passiert das distale Ende 45 des Montageelements 5 den Übergangsbereich 21, so bewegen sich die erste 50 und die zweite Hälfte 51 durch die elastische Ausgestaltung aufeinander zu, so dass schlussendlich das Montageelement 5 und somit die Sicherheitseinrichtung 2 auf dem Spritzenkörper 1 aufgeclipst ist.

Zur Sicherung dieser Clipverbindung weisen die Rastelemente 48 an ihrem distalen Ende 45 Sicherungsabschnitte 52 auf, die sich in Umfangsrichtung eines Innenkreises 53 des Montageelements 5 erstrecken und mit dem Übergangsbereich 21 in mechanischen Wirkkontakt stehen.

Erfindungsgemäß ist die Sicherheitseinrichtung 2 umfassend die Hülse 2, das Montageelement 5, das Federelement 4 und gegebenenfalls die Kappe 9 bereits vormontierbar und als Ganzes mit dem Spritzenkörper 1 mittels des Montageelements 5 verbindbar.

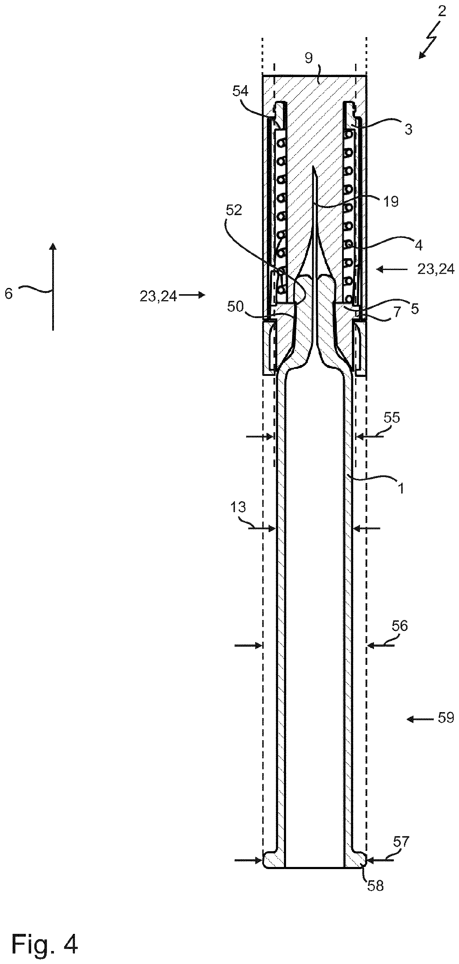

Die Figur 4 zeigt einen Spritzenkörper 1 mit einer darauf montierten Sicherheitseinrichtung 2 in einem Längsschnitt.

Die Sicherheitseinrichtung 2 zeigt nun zusätzlich zu der Kappe 9, der Hülse 22 und dem Montageelement 5 auch das Federelement 4, das vorliegend als eine Spiralfeder 4 ausgebildet ist.

Wie deutlich zu erkennen ist, stehen die Führungsstifte 7 mit der Ausnehmung 24 bzw. der Führungskulisse 23 in Kontakt, so dass die Führungsstifte 7 durch die Führungskulisse 23 bei einer Relativbewegung des Spritzenkörpers 1 gegenüber der Sicherheitseinrichtung 2 geführt werden.

Das Montageelement 5 ist mit dem verengten Bereich 15 mittels der Rastelemente 48 und deren Sicherungsabschnitte 52 miteinander verclipst und mittels der Führungsstifte 7 mit der Hülse 22 verbunden.

Die Hülse 22 umfasst weiter an seinem distalen Ende 27 einen innenliegenden Auflagebereich 54, der mit dem Federelement 4 wirkverbindbar ist. Das Federelement 4 wird also einerseits durch den Auflagebereich 54 und andererseits durch das Montageelement 5 in der Hülse 22 gehalten und wird so gegen ein Herausfallen gesichert.

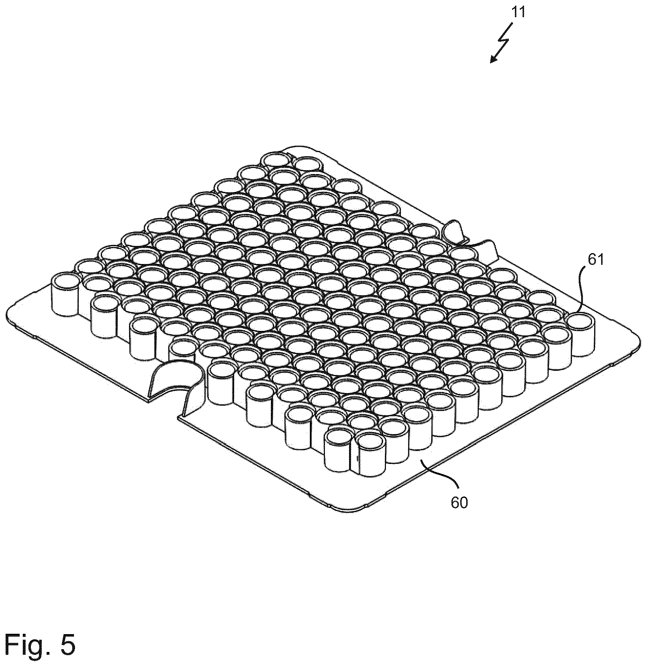

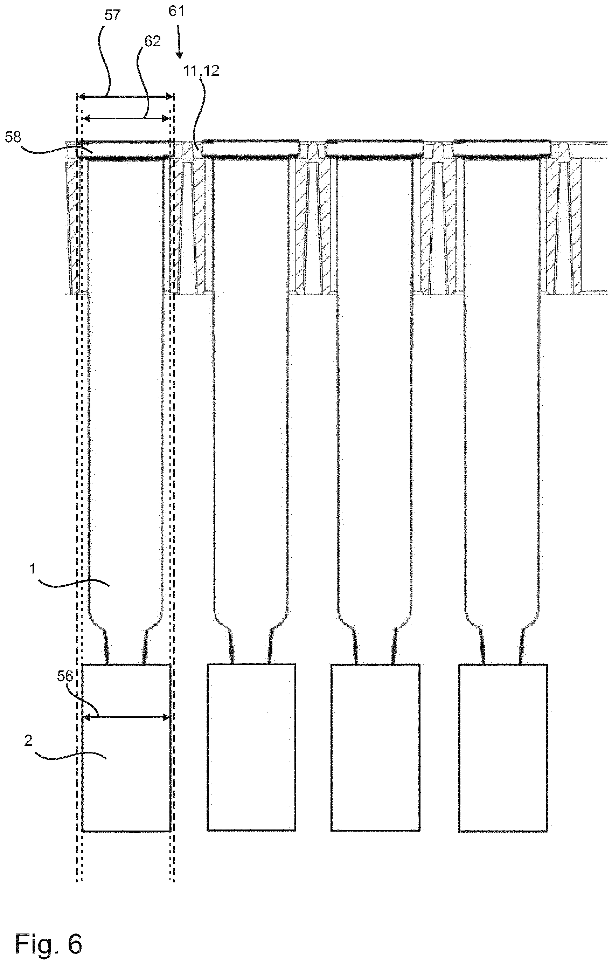

Die Dimensionen der Sicherheitseinrichtung 2 sind derart gewählt, dass die Hülse 22 einen Innendurchmesser 55 aufweist, der größer ist als der Durchmesser 13 des Spritzenkörpers 1, so dass der Spritzenkörper 1 bei einer Bewegung in Längsrichtung L nach vorne, wobei die Bewegungsrichtung gekennzeichnet ist durch einen Pfeil L, gegenüber der Sicherheitseinrichtung 2 der Spritzenkörper in die Hülse 22 hineinbewegbar ist. Gleichzeitig ist der Außendurchmesser 56 der Hülse 22 bzw. der Sicherheitseinrichtung 2 so gewählt, dass er höchstens einem maximalen Durchmesser 57 einer am proximalen Ende 59 des Spritzenkörpers 1 angebrachten Haltevorrichtung 58 zum Halten und sicheren Setzen der Spritze entspricht. Der Zweck dieser Größeneinschränkung wir mit Bezug auf Figuren 5 und 6 genauer dargestellt.

Figur 5 zeigt dabei ein Spritzennest, wie es schon aus dem Stand der Technik bekannt ist, umfassend eine Grundplatte 60, auf dem Hohlzylinder 61, also insbesondere Öffnungen 61, angeordnet sind, in welche die Spritzen eingehängt werden können. Die Grundplatte 60 dient hierbei dazu, das Spritzennest in einer Wanne 12 (hier nicht gezeigt) zu befestigen.

Die Figur 6 zeigt hierzu einen Ausschnitt eines Längsschnitts eines Spritzennests 11 mit eingehängten Spritzen, wobei die Spritzen einen Spritzenkörper 1 und eine Sicherheitseinrichtung 2 umfassen. Dabei ist der Spritzenkörper 1 mit der Sicherheitseinrichtung 2 zusammen in das Spritzennest eingehängt worden. Um eine Spritze in das Spritzennest 11 einhängen zu können, ist der Durchmesser 57 der Haltevorrichtung 58 größer gewählt als der Durchmesser 62 einer Öffnung 61. Um den Spritzenkörper 1 samt Sicherheitseinrichtung 2 in eine Öffnung 61 einhängen zu können, muss also der Außendurchmesser 56 der Hülse 22 bzw. der Außendurchmesser 56 der Sicherheitseinrichtung 2 kleiner gewählt sein als der Durchmesser 57 der Haltevorrichtung 58. Es ist daher möglich, die Spritze bereits mit der Sicherheitseinrichtung 2 in das Spritzennest einzuhängen, zu sterilisieren und zu befüllen.

Anschließend kann das Spritzennest 11 umfassend Spritzenkörper 1, wobei das Spritzennest 11 in der Wanne 12 eingehängt ist, mittels einer Bedeckung, insbesondere einer Folie und/oder einer Membran versiegelt werden bzw. verschlossen werden. Die Folie ist beispielsweise aus Kunststoff hergestellt bzw. bei der Membran handelt es sich um eine Tyvek-Folie. Nach dem Versiegeln bzw. dem Verschließen der Wanne 12 wird die vorliegende Anordnung sterilisiert.

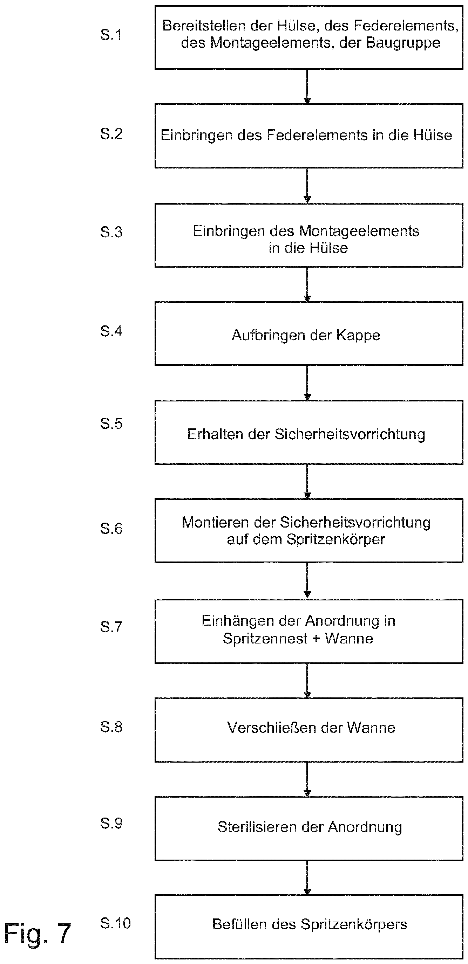

Die Figur 7 gibt dabei einen Verfahrensablauf gemäß einer besonders bevorzugten Ausführungsform wieder.

In einem ersten Schritt S.1 werden die jeweiligen Elemente, die Hülse 3, das Federelement 4, das Montageelement 5 und die Baugruppe 8 umfassend Kappe 9 und Nadelschutzteil 10, bereitgestellt. Bei diesen Elementen handelt es sich vorzugsweise um vorgefertigte Bauteile, die in hoher Stückzahl produziert werden.

Im Schritt S.2 wird das Federelement 4 in das Innere der Hülse 3 eingebracht und in einem Schritt S.3 durch das Einbringen des Montageelements 5 in das Innere der Hülse 3 gegen ein Herausfallen gesichert.

In einem darauf folgenden Schritt S.4 wird auf die Hülse 3 eine Baugruppe 8, insbesondere die Kappe 9, auf die Hülse 3 aufgeschoben, so dass in einem Schritt S.5 die Sicherheitseinrichtung 2 vormontiert ist und erhalten wird.

Die erhaltene Sicherheitseinrichtung 2 wird in einem Schritt S.6 in einem einzigen Schritt auf dem Spritzenkörper 1 montiert, indem die Sicherheitseinrichtung 2 an dem distalen Ende des Spritzenkörpers 1 aufgeschoben wird und somit das Montageelement 5 mit dem Spritzenkörper 1 verbunden wird.

Nach Montage der Sicherheitseinrichtung 2 auf dem Spritzenkörper 1 kann die gesamte Anordnung in einem Schritt S.7 in ein Spritzennest 11 und eine Wanne 12 eingehängt werden und in einem Schritt S.8 die Wanne 12 verschlossen werden, etwa mit einer Schutzfolie und/oder einer Membran und vorzugsweise mit einer Tyvek-Folie.

In einem darauf folgenden Schritt S.9 wird die Anordnung bestehend aus Spritzenkörper 1 mit Sicherheitseinrichtung 2 und Wanne 12 mit Spritzennest 11 sterilisiert werden, vorzugsweise unter Reinraumbedingungen.

In einem letzten Schritt S.10 wird vorzugsweise die Folie wieder entfernt, so dass ein Befüllen der Spritzenkörper 1 durchgeführt werden kann. In einem nicht gezeigten nachfolgenden Schritt werden die befüllten Spritzenkörper 1 mit Stopfen oder dergleichen versehen und dadurch verschlossen und anschließend in handelsübliche Verpackungen gegeben.

### Bezugszeichenliste

- 1: Spritzenkörper
- 2: Sicherheitseinrichtung
- 3: Hülse
- 4: Federelement
- 5: Montageelement
- 6: Montagerichtung
- 7: Führungsstift
- 8: Baugruppe
- 9: Kappe
- 10: Nadelschutzteil
- 11: Spritzennest
- 12: Wanne
- 13: Durchmesser Spritzenkörper
- 14: distales Ende Spritzenkörper
- 15: verengter Bereich
- 16: Durchmesser verengter Bereich
- 17: distales Ende verengter Bereich
- 18: Nadelhalterung
- 19: Stechelement
- 20: Durchmesser Nadelhalterung
- 21: Übergangsbereich
- 22: Hülse
- 23: Führungskulisse
- 24: Ausnehmung
- 25: Ausnahmebereich
- 26: Berandung
- 27: distales Ende Hülse
- 28: Kreisring
- 29: ringförmiger Bereich
- 30: Austrittsöffnung
- 31: proximales Ende Hülse
- 32: schräger Abschnitt
- 33: Übergang
- 34: Hülseninnenwand
- 35: Wandstärke
- 36: Betrag Vertiefung
- 37: distales Ende der Kappe
- 38: proximales Ende der Kappe
- 39: erstes Flügelelement
- 40: zweites Flügelelement
- 41: Innendurchmesser
- 42: kreisförmiger Rand
- 43: Außendurchmesser Montageelement
- 44: Innendurchmesser Montageelement
- 45: distales Ende Montageelement
- 46: Mantelfläche
- 47: Aussparung
- 48: Rastelement
- 49: proximales Ende Montageelement
- 50: erste Hälfte
- 51: zweite Hälfte
- 52: Sicherungsabschnitt
- 53: Innenkreis
- 54: Auflagebereich
- 55: Innendurchmesser Hülse
- 56: Außendurchmesser Hülse
- 57: maximaler Durchmesser
- 58: Haltevorrichtung
- 59: proximales Ende Spritzenkörper
- 60: Grundplatte
- 61: Öffnung
- 62: Durchmesser Öffnung

## Patentansprüche

1. Verfahren zur Montage einer Sicherheitseinrichtung (2) und Montage der Sicherheitseinrichtung (2) auf einem Spritzenkörper (1) vor einem Abfüllprozess des Spritzenkörpers (1), umfassend die Schritte:
a. Bereitstellen einer Hülse (3; 22), eines Federelements (4) und eines Montageelements (5),
b. Einbringen des Federelements (4) in das Innere der Hülse (3; 22) entlang einer Montagerichtung (6),
c. Einbringen des Montageelements (5) in das Innere der Hülse (3; 22) entlang der Montagerichtung (6),
d. Erhalten der Sicherheitseinrichtung (2),
e. Montieren der Sicherheitseinrichtung (2) auf dem Spritzenkörper (1) vor dem Abfüllprozess des Spritzenkörpers (1) durch Verbinden des Montageelements (5) mit dem Spritzenkörper (1);
f. Einhängen des Spritzenkörpers (1) mit der montierten Sicherheitseinrichtung (2) in ein Spritzennest (11) zum nachträglichen Befüllen des Spritzenkörpers (1);
wobei auf dem Montageelement (5) mindestens ein Führungsstift (7) angeordnet ist, und die Hülse (22) mindestens eine Ausnehmung (24), welche eine Führungskulisse (23) zur Führung des Führungsstiftes (7) ausbildet, und an einem proximalen Ende (31) zumindest einen schrägen Abschnitt (32) aufweist, welcher eine Vertiefung der Hülseninnenwand (34) darstellt und komplementär zu dem mindestens einen Führungsstift (7) ausgebildet ist,
und im Schritt c. das Montageelement (5) mit der Hülse (3; 22) mittels des mindestens einen Führungsstifts (7) verbunden wird, wobei der Führungsstift (7) durch den schrägen Abschnitt (32) und durch die Führungskulisse (23) geführt wird,
wobei der Führungsstift (7) bei einer Relativbewegung des Spritzenkörpers (1) zur Sicherheitseinrichtung (2) geführt wird, wobei das Montageelement (5) hierzu um eine Achse des Spritzenkörpers (2) rotiert und sich in axialer Richtung des Spritzenkörpers (2) nicht bewegt,
**dadurch gekennzeichnet, dass**
zunächst die Sicherheitseinrichtung (2) zusammengebaut und anschließend als Einheit in einem einzigen Schritt auf dem Spritzenkörper (1) montiert wird.

2. Verfahren nach Anspruch 1,
**dadurch gekennzeichnet, dass**
das Montageelement (5) mit dem Spritzenkörper (1) durch Aufstecken des Montageelements (5) auf dem Spritzenkörper (1) verbunden wird.

3. Verfahren nach Anspruch 2,
**dadurch gekennzeichnet, dass**
das Montageelement (5) auf dem Spritzenkörper (1) aufgeclipst wird.

4. Verfahren nach einem der vorhergehenden Ansprüche 1 bis 3,
**dadurch gekennzeichnet, dass**
zusätzlich eine Baugruppe (8) umfassend eine Kappe (9) und ein Nadelschutzteil (10) bereitgestellt wird.

5. Verfahren nach Anspruch 4,
**dadurch gekennzeichnet, dass**
die Baugruppe (8) mit der Hülse (3; 22) durch Aufschieben der Kappe (9) auf der Hülse (3; 22) entgegen der Montageeinrichtung (6) verbunden wird.

6. Verfahren nach einem der vorhergehenden Ansprüche 1 bis 5,
**dadurch gekennzeichnet, dass**
der Spritzenkörper (1) mit der montierten Sicherheitseinrichtung (2) sterilisiert wird.

7. Verfahren nach einem der vorhergehenden Ansprüche 1 bis 6,
**dadurch gekennzeichnet, dass**
das Spritzennest (11) in eine Wanne (12) eingehängt wird.

## Claims

1. Method for mounting a safety device (2) and for mounting the safety device (2) on a syringe body (1) before the syringe body (1) is filled, comprising the steps of:
a. providing a sleeve (3; 22), a spring element (4) and a mounting element (5),
b. inserting the spring element (4) into the interior of the sleeve (3; 22) along a mounting direction (6),
c. inserting the mounting element (5) into the interior of the sleeve (3; 22) along the mounting direction (6),
d. obtaining the safety device (2),
e. mounting the safety device (2) on the syringe body (1) before the syringe body (1) is filled by connecting the mounting element (5) to the syringe body (1);
f. mounting the syringe body (1) together with the safety device (2) mounted thereon in a syringe nest (11) for subsequent filling of the syringe body (1);
at least one guide pin (7) being arranged on the mounting element (5) and the sleeve (22) comprising at least one recess (24), which forms a guide slot (23) for guiding the guide pin (7), and comprises at least one slanting portion (32) at a proximal end (31), which constitutes a depression in the inner wall (34) of the sleeve and is designed complementarily to the at least one guide pin (7), and, in step c., the mounting element (5) being connected to the sleeve (3; 22) by means of the at least one guide pin (7), the guide pin (7) being guided by the slanting portion (32) and by the guide slot (23),
the guide pin (7) being guided in a movement of the syringe body (1) relative to the safety device (2), the mounting element (5), for this purpose, rotating about an axis of the syringe body (2) and not moving in the axial direction of the syringe body (2),
**characterised in that**
the safety device (2) is first assembled and then mounted on the syringe body (1) in a single step as a unit.

2. Method according to claim 1,
**characterised in that**
the mounting element (5) is connected to the syringe body (1) by the mounting element (5) being placed on the syringe body (1).

3. Method according to claim 2,
**characterised in that**
the mounting element (5) is clipped onto the syringe body (1).

4. Method according to any of the preceding claims 1 to 3,
**characterised in that**
an assembly (8) comprising a cap (9) and a needle guard (10) is additionally provided.

5. Method according to claim 4,
**characterised in that**
the assembly (8) is connected to the sleeve (3; 22) by pushing the cap (9) onto the sleeve (3; 22) counter to the mounting device (6).

6. Method according to any of the preceding claims 1 to 5,
**characterised in that**
the syringe body (1) together with the safety device (2) mounted thereon is sterilised.

7. Method according to any of the preceding claims 1 to 6,
**characterised in that**
the syringe nest (11) is mounted in a trough (12).

## Revendications

1. Procédé de montage d'un dispositif de sécurité (2) et pour le montage du dispositif de sécurité (2) sur un corps de seringue (1) avant que le corps de seringue (1) ne soit rempli, comportant les étapes :
a. fourniture d'un manchon (3 ; 22), d'un élément ressort (4) et d'un élément de montage (5),
b. introduction de l'élément ressort (4) dans l'intérieur du manchon (3 ; 22) le long d'une direction de montage (6),
c. introduction de l'élément de montage (5) dans l'intérieur du manchon (3 ; 22) le long de la direction de montage (6),
d. obtention du dispositif de sécurité (2),
e. montage du dispositif de sécurité (2) sur le corps de seringue (1) avant que le corps de seringue (1) ne soit rempli par liaison de l'élément de montage (5) avec le corps de seringue (1) ;
f. accrochage du corps de seringue (1) avec le dispositif de sécurité monté (2) dans un nid de seringue (11) pour remplissage ultérieure du corps de seringue (1) ;
dans lequel, sur l'élément de montage (5), au moins une broche de guidage (7) est disposée, et le manchon (22) présente au moins une cavité (24), laquelle forme une coulisse de guidage (23) pour le guidage de la broche de guidage (7), et présente, à une extrémité proximale (31), au moins une partie inclinée (32), laquelle constitue un évidement de la paroi interne de manchon (34) et est formée de façon complémentaire à ladite au moins une broche de guidage (7),
et, à l'étape c., l'élément de montage (5) est relié au manchon (3 ; 22) au moyen de ladite au moins une broche de guidage (7), la broche de guidage (7) étant guidée par la partie inclinée (32) et par la coulisse de guidage (23), dans lequel la broche de guidage (7) est guidée lors d'un mouvement relatif du corps de seringue (1) vers le dispositif de sécurité (2), l'élément de montage (5) tournant à cet effet autour d'un axe du corps de seringue (2) et ne se déplaçant pas dans la direction axiale du corps de seringue (2)
**caractérisé par le fait que**
d'abord le dispositif de sécurité (2) est assemblé puis est monté sur le corps de seringue (1) en tant qu'unité en une seule étape.

2. Procédé selon la revendication 1,
**caractérisé par le fait que**
l'élément de montage (5) est relié au corps de seringue (1) par enfichage de l'élément de montage (5) sur le corps de seringue (1).

3. Procédé selon la revendication 2,
**caractérisé par le fait que**
l'élément de montage (5) est clipsé sur le corps de seringue (1).

4. Procédé selon l'une des revendications précédentes 1 à 3,
**caractérisé par le fait qu'**
il comporte en outre un ensemble (8) comportant un capuchon (9) et une partie protectrice d'aiguille (10).

5. Procédé selon la revendication 4,
**caractérisé par le fait que**
l'ensemble (8) est relié au manchon (3 ; 22) par poussée du capuchon (9) sur le manchon (3 ; 22) contre le dispositif de montage (6).

6. Procédé selon l'une des revendications précédentes 1 à 5,
**caractérisé par le fait que**
le corps de seringue (1) est stérilisé avec le dispositif de sécurité monté (2).

7. Procédé selon l'une des revendications précédentes 1 à 6,
**caractérisé par le fait que**
le nid de seringue (11) est accroché dans un bac (12).
